# EUROPÄISCHE PATENTSCHRIFT

(11) **EP 0 852 934 B1**
(45) Veröffentlichungstag und Bekanntmachung des Hinweises auf die Patenterteilung: **31.07.2002**
(21) Anmeldenummer: 97810009.7
(22) Anmeldetag: 10.01.1997
(51) Int. Cl.: A61F 2/38

(54) **Tibiaplattform für ein künstliches Kniegelenk**
Tibial tray for an artificial knee joint
Plateau tibial pour une articulation artificielle du genou

(43) Veröffentlichungstag der Anmeldung: 15.07.1998
(73) Patentinhaber: Sulzer Orthopädie AG, 6340 Baar (CH)
(72) Erfinder: Buni, Richard, 8442 Hettlingen (CH)
(74) Vertreter: Manitz, Finsterwald & Partner Gbr

(56) Entgegenhaltungen:
- EP-A- 0 381 352
- EP-A- 0 738 504
- DE-C- 3 730 175
- FR-A- 2 720 267
- US-A- 5 152 797
- US-A- 5 330 534

## Beschreibung

Die Erfindung handelt von einer Tibiaplattform für ein künstliches Kniegelenk mit einem metallischen Unterteil, das in der Tibia verankerbar ist, mit einem Lagerteil aus Kunststoff, welches Gleitflächen für zwei Femurkondylen aufweist, welches über ein offenes Scharniergelenk posterior am Unterteil einschwenkbar ist, und welches in einem Abstand "l" vom Scharniergelenk weg gegen anterior an einem Vorsprung des Unterteils mit einer Klinke verankerbar ist.

Eine derartige Tibiaplattform ist in der Patentanmeldung EP-A-0 738 504 gezeigt. Eine einstückig an dem Lagerteil angeformte Klinke rastet hier bei der Montage unter dem Vorsprung des Unterteils ein. Solche Lagerteile werden häufig in Kunststoff beispielsweise in Polyethylen ausgeführt, um eine günstige Gleitpaarung zu metallischen Femurkondylen zu erreichen. Die verwendeten Kunststoffe haben den Vorteil, dass sie mit ihrer Struktur und Festigkeit hohe Punktlasten vermeiden. Das heisst bei einer Verklinkung sind diesen Kunststoffen Grenzen gesetzt.

Bei den Kräften, die auf das Lagerteil und weiter auf das Unterteil der Tibiaplattform wirken hängen die Richtung und die maximalen Beträge stark davon ab, wie intakt der Bandapparat noch ist. Je weniger von den Bändern noch wirksam sind, desto mehr müssen die Femurkondylen durch eine mittlere Führungsrippe in Form einer künstlichen Eminentia geführt werden und desto extremer sind die Belastungen, die an dem Lagerteil angreifen.

Eine weitere Verriegelung einer Tibiaplattform mit einem Lagerteil ist in der Patentschrift US 5,330,534 gezeigt. Aus einer ebenen Oberfläche der Plattform ragen posterior und anterior in der Mitte Vorsprünge hervor. Auf der posterioren Seite wird das Lagerteil mit einem offenen Scharnier gegen Abheben gesichert. Anterior sind zwei vorstehende Stifte vorgesehen, die quer verlaufende Aussparungen aufweisen, um mit einem Riegel, der durch das Lagerteil und diese Aussparungen verläuft, das Lagerteil gegen ein Abheben anterior zu sichern. Der Riegel selbst ist mit einer Schnappfeder gegen Herausfallen gesichert. Der Riegel und die vorstehenden Stifte sind aus Metall, müssen aber mit Spiel zueinander montierbar sein. Kräfte, die vom Lagerteil auf den Riegel und weiter auf die vorstehenden Stifte übertragen werden, führen zwangsweise zu Mikrobewegungen und zu Metallabrieb zwischen Riegel und Stiften. Sämtliche Querkräfte zwischen Lagerteil und Plattform werden im Bereich der Vorsprünge posterior und anterior übertragen. Ein weiterer Nachteil besteht darin, dass durch den Verriegelungsmechanismus anterior an der Aussenseite des Lagerteils zusätzliche Ecken und Kanten bestehen, die zu Quetschungen von Gewebeteilen führen, wenn der Träger einer solchen Prothese vorn das Knie anschlägt.

Aufgabe der Erfindung ist für diese Fälle eine wirksame und dauerhafte Verankerung des Lagerteils im Unterteil zu schaffen. Diese Aufgabe wird mit den Kennzeichen vom unabhängigen Anspruch 1 gelöst.

Die Anordnung hat den Vorteil, dass auch Varus- und Valgusstellungen des Kniegelenks bei schwachem Bandapparat aufgefangen werden. Ein weiterer Vorteil besteht darin, bei einem bereits in der Tibia eingesetzten Unterteil trotz der vorstehenden Eminentia eine raumsparendes Einsetzen des Lagerteils von anterior her möglich ist. Das Lagerteil ist in einem sehr flachen Winkel in das offene Scharniergelenk von anterior her einschiebbar, während die Hilfsmittel zur Verankerung der metallischen Klinke am Lagerteil von oben und von anterior angebracht werden können. Ein weiterer Vorteil besteht darin, dass für ein bereits implantiertes Unterteil im Sinne eines Baukastens Lagerteile mit und ohne ausgeprägte Führungsrippe und mit unterschiedlichem Abstand der Lagerfläche zum Unterteil einsetzbar sind. Ein Operateur kann sich daher nach dem Einsetzen des Unterteils entsprechend dem Zustand des Bandapparates für ein passendes Lagerteil entscheiden.

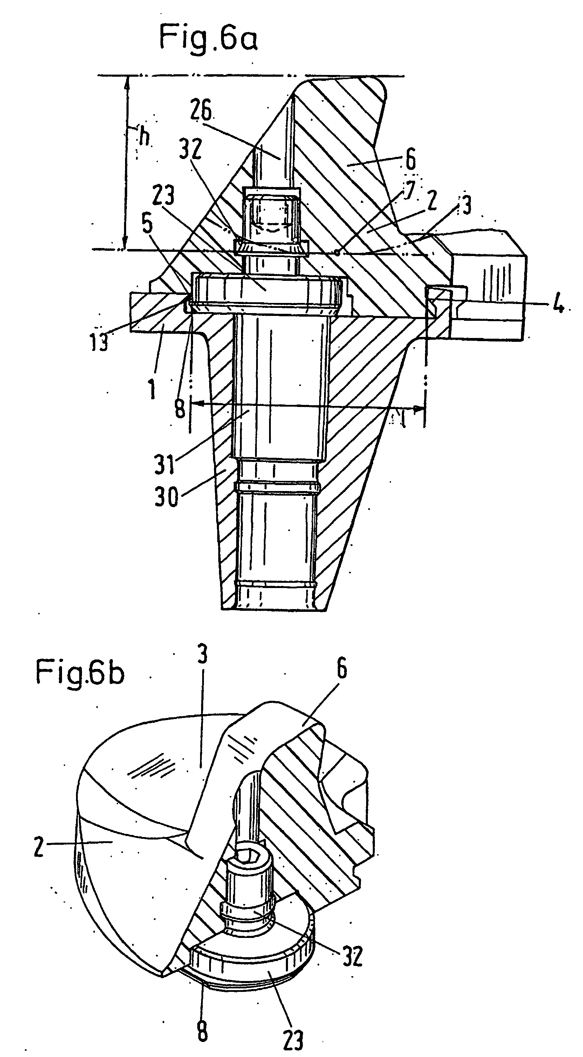

Vorteilhafte Weiterbildungen der Erfindung sind in den abhängigen Ansprüchen 2 bis 6 gezeigt. Im folgenden wird die Erfindung an einem Ausführungsbeispiel beschrieben.
Es zeigen:
- Fig. 6a: schematisch einen Schnitt in sagittaler Richtung durch ein Unterteil und ein Lagerteil, welche durch eine metallische Klinke an der Peripherie eines im Lagerteil verankerten Drehbolzens verbunden sind;
- Fig. 6b: Schematisch eine Ansicht von Lagerteil und Drehbolzen in Figur 6a; und

Die Figuren zeigen eine Tibiaplattform mit metallischem Unterteil 1 und mit verschiedenen Lagerteilen 2 aus Kunststoff, die über ein offenes Scharniergelenk 4 posterior miteinander verankerbar sind. Das Lagerteil 2 besitzt eine Führungsrippe 6 zur Führung von Femurkondylen, die mindestens 15 mm über den tiefsten Punkt 7 der Gleitflächen 3 nach oben herausragt. Dadurch, dass an einem horizontalen Vorsprung 5 des Unterteils 1 in einem Abstand "l" zum Scharniergelenk 4 eine separate metallische Klinke 8 eingreift, die zum Lagerteil 2 verschiebbar gelagert ist und die mit Hilfsmitteln 9 durch eine erzwungene Bewegung in einer Einraststellung 13 am Vorsprung 5 dauerhaft positionierbar ist, können grosse Seitenkräfte, wie sie bei hohen Führungsrippen 6 und Varus-/Valgusstellungen des Knies vorkommen, aufgefangen werden.

In den Figuren 6a, b ist im Lagerteil 2 in vertikaler Richtung ein Drehbolzen 23 verankert, der auf der unteren Seite zu einer Scheibe erweitert ist und der von oben durch eine Lippe 32 gegen Herausfallen gesichert ist. Der Drehbolzen endet als Innensechskant, das durch eine Durchgangsbohrung 26 von oben mit einem Schlüssel erreichbar ist. Die Klinke 8 ist als exzentrische Schulter an der Peripherie des Drehbolzens 23 angeformt und kann mit dem Schlüssel zur Verklinkung unter den Vorsprung 5 gedreht werden. Eine Selbsthemmung der Klinke 8 wird beispielsweise dadurch erreicht, dass sie entlang dem Umfang leicht ansteigt, um sich selbst unter dem Vorsprung 5 zu verkeilen.

## Patentansprüche

1. Tibiaplattform für ein künstliches Kniegelenk mit einem metallischen Unterteil (1), das in der Tibia verankerbar ist, mit einem Lagerteil (2) aus Kunststoff, welches Gleitflächen (3) für zwei Femurkondylen aufweist, welches über ein offenes Scharniergelenk (4) posterior am Unterteil (1) einschwenkbar ist, und welches in einem Abstand "l" vom Scharniergelenk (4) weg gegen anterior an einem Vorsprung (5) des Unterteils mit einer Separaten metallischen Klinke (8) verankerbar ist, wobei die Klinke (8) zum Lagerteil (2) verschiebbar gelagert ist und dass die Klinke (8) durch eine erzwungene Bewegung mit Hilfsmitteln in einer Einraststellung (13) am Vorsprung (5) dauerhaft positionierbar ist, **dadurch gekennzeichnet, dass** die Klinke (8) als Schulter an der Peripherie eines im Lagerteil (2) verankerten Drehbolzen (23) ausgeführt ist, wobei die Klinke (8) durch Drehung des in axialer Richtung gesicherten Drehbolzens (23) unter dem Vorsprung (5) einfahrbar ist.

2. Tibiaplattform nach Anspruch 1, **dadurch gekennzeichnet, dass** die axiale Sicherung des Drehbolzens (23) gegen Herausfallen durch eine Lippe (32) des Drehbolzens realisiert ist.

3. Tibiaplattform nach einem der Ansprüche 1 oder 2, **dadurch gekennzeichnet, dass** der Drehbolzen (23) auf seiner unteren Seite als Scheibe erweitert ist, an deren Peripherie die Klinke (8) als exzentrische Schulter angeformt ist.

4. Tibiaplattform nach Anspruch 3, **dadurch gekennzeichnet, dass** die Klinke (8) entlang dem Umfang leicht ansteigt, um sich selbst unter dem Vorsprung (5) zur Selbsthemmung zu verkeilen.

5. Tibiaplattform nach einem der Ansprüche 1 bis 4, **dadurch gekennzeichnet, dass** der Drehbolzen (23) als Innensechskant endet, der durch eine Durchgangsbohrung (26) im Lagerteil (2) von oben mit einem Schlüssel als Hilfsmittel erreichbar ist, um Drehbolzen (23) und Klinke (8) zu drehen.

6. Tibiaplattform nach einem der Ansprüche 1 bis 5, bei der zwischen den Gleitflächen (3) eine Führungsrippe (6) mindestens mit einer Höhe h = 15 mm über einen tiefsten Punkt (7) der Gleitfläche (3) hochsteht.

## Claims

1. Tibia platform for an artificial knee joint compris ing a metallic lower part (1) which can be anchored in the tibia and a bearing part (2) which is made of plastic, which has sliding surfaces (3) for two femur condyles, which can be pivoted at the posterior at the lower part (1) via an open hinge joint (4) and which can be anchored at a distance "1" away from the hinge joint in the anterior direction at a projection (5) of the lower part by a separate metallic pawl (8), with the pawl (8) being displaceably mounted relative to the bearing part (2) and being capable of being permanently positioned in a latching position (13) at the projection (5) by an enforced movement by auxiliary means (9), **characterised in that** the pawl (8) is formed as a shoulder (22) at the periphery of a rotatable pin (23) which is anchored in the bearing part (2), with the pawl (8) being engageable under the projection (5) by rotation of the rotatable pin (23), which is secured in the axial direction.

2. Tibia platform in accordance with claim 1, **characterised in that** the axial securing of the rotatable pin (23) against dropping out is realized by a lip (32) of the rotatable pin.

3. Tibia platform in accordance with claim 1 or claim 2, **characterised in that** the rotatable pin (23) is enlarged at its lower side as a disc, with the pawl (8) being formed as an eccentric shoulder at its periphery.

4. Tibia platform in accordance with claim 3, character ised in that the pawl (8) rises slightly along the periphery in order to wedge itself by self locking under the projection (5).

5. Tibia platform in accordance with one of the claims 1 to 4, **characterised in that** the rotatable pin (23) ends in an internal hexagon which is accessible with a key as an auxiliary means from above through a through bore (26) in the bearing part, in order to rotate the rotatable pin and the pawl (8).

6. Tibia platform in accordance with one of the claims 1 to 5, in which a guide rib (6) projects between the sliding surfaces(3), with a height h at least equal to 15mm above a lowest point (7) of the sliding surface.

## Revendications

1. Plateau tibial pour une articulation artificielle du genou avec une partie inférieure métallique (1) qui peut être ancrée dans le tibia, avec une partie de palier (2) en matière synthétique qui présence des faces de glissement (3) pour deux condyles de fémur, qui peut être pivotée vers l'intérieur par une articulation en charnière ouverte (4) postérieurement à la partie inférieure (1) et qui peut être ancrée à un écart "1" de l'articulation en charnière (4) contre le côté antérieur à une saillie (5) de la partie inférieure avec un cliquet métallique séparé (8), où le cliquet (8) est logé d'une manière déplaçable relativement à la partie de palier (2), et en ce que le cliquet (8) peut être positionné d'une manière durable par un mouvement forcé avec des moyens auxiliaires dans une position d'enclenchement (13) à la saillie (5), **caractérisé en ce que** le cliquet (8) est réalisé sous forme d'épaulement à la périphérie d'un boulon tournant (23) ancré dans la partie de palier (2), où le cliquet (8) peut être rentré par une rotation du boulon tournant (23) assuré dans la direction axiale sous la saillie (5).

2. Plateau tibial selon la revendication 1, **caractérisé en ce que** la protection axiale du boulon tournant (23) à l'encontre d'une chute est réalisée par une lèvre (32) du boulon tournant.

3. Plateau tibial selon l'une des revendications 1 ou 2, **caractérisé en ce que** le boulon tournant (23) est élargi à son côté inférieur en disque à la périphérie duquel est rapporté par formage le cliquet (8) comme épaulement excentré.

4. Plateau tibial selon la revendication 3, **caractérisé en ce que** le cliquet (8) remonte légèrement le long du pourtour pour se caler lui-même sous la saillie (5) en vue d'un auto-blocage.

5. Plateau tibial selon l'une des revendications 1 à 4, **caractérisé en ce que** le boulon tournant (23) se termine comme six pans creux qui peut être atteint à travers un perçage traversant (26) dans la partie de palier (2) de dessus avec une clé, comme moyen auxiliaire pour faire tourner le boulon tournant (23 )et le cliquet (8).

6. Plateau tibial selon l'une des revendications 1 à 5, où une nervure de guidage (6) s'élève entre les faces de glissement (3) au moins avec une hauteur h = 15 mm sur un point le plus bas (7) de la face de glissement (3).
